# EUROPÄISCHE PATENTSCHRIFT

(11) **EP 2 861 633 B1**
(45) Veröffentlichungstag und Bekanntmachung des Hinweises auf die Patenterteilung: **10.08.2016**
(21) Anmeldenummer: 13727895.8
(22) Anmeldetag: 10.06.2013
(51) Int. Cl.: B07B 1/12, C08F 6/00, A61L 15/60

(54) **VERFAHREN ZUR HERSTELLUNG WASSERABSORBIERENDER POLYMERPARTIKEL**
METHOD FOR THE PRODUCTION OF WATER-ABSORBING POLYMER PARTICLES
PROCÉDÉ DE PRÉPARATION DE PARTICULES POLYMÈRES ABSORBANT L'EAU

(30) Priorität: 19.06.2012 EP 12172596
(43) Veröffentlichungstag der Anmeldung: 22.04.2015
(73) Patentinhaber: BASF SE, 67056 Ludwigshafen am Rhein (DE)
(72) Erfinder: KRAUß, Roland, 67435 Neustadt (DE); GIEGER, Thomas, 67069 Ludwigshafen (DE); KLOCK, Volker, 67061 Ludwigshafen (DE)
(74) Vertreter: BASF IP Association
(86) Internationale Anmeldenummer: PCT/EP2013/061901
(87) Internationale Veröffentlichungsnummer: WO 2013/189770

(56) Entgegenhaltungen:
- WO-A1-89/09090
- JP-A- H06 107 800
- Anonym: "Stangensizer", Mogensen-ALLGAIER-Gruppe , 24. September 2006 (2006-09-24), XP002713553, Gefunden im Internet: URL:http://www.mogensen.de/de/stangensizer .htm [gefunden am 2013-09-25]
- Anonym: "Vibro-Stangensizer", Mogensen-ALLGAIER-Gruppe , 25. Oktober 2005 (2005-10-25), XP002713554, Gefunden im Internet: URL:http://www.mogensen.de/de/vibrostangen sizer.htm [gefunden am 2013-09-25]

## Beschreibung

Die vorliegende Erfindung betrifft ein Verfahren zur Herstellung wasserabsorbierender Polymerpartikel, umfassend Polymerisation einer Monomerlösung oder -suspension, Klassierung und Trocknung des erhaltenen feuchten Polymergels, sowie Zerkleinerung des bei der Klassierung des feuchten Polymergels abgetrennten Grobanteils, wobei die Klassierung des feuchten Polymergels mittels mehrerer kaskadenförmig angeordneter Stangendecks in einem Schwingrahmen durchgeführt wird.

Wasserabsorbierende Polymerpartikel werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden auch als Superabsorber bezeichnet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Die Eigenschaften der wasserabsorbierenden Polymerpartikel können beispielsweise über die verwendete Vernetzermenge eingestellt werden. Mit steigender Vernetzermenge sinkt die Zentrifugenretentionskapazität (CRC) und die Absorption unter einem Druck von 21,0 g/cm² (AUL0.3psi) durchläuft ein Maximum.

Zur Verbesserung der Anwendungseigenschaften, wie beispielsweise Permeabilität des gequollenen Gelbetts (SFC) in der Windel und Absorption unter einem Druck von 49.2 g/cm² (AUL0.7psi), werden wasserabsorbierende Polymerpartikel im allgemeinen oberflächennachvernetzt. Dadurch steigt der Vernetzungsgrad der Partikeloberfläche, wodurch die Absorption unter einem Druck von 49,2 g/cm² (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet und thermisch oberflächennachvernetzt. Dazu geeignete Vernetzer sind Verbindungen, die mit mindestens zwei Carboxylatgruppen der wasserabsorbierenden Polymerpartikel kovalente Bindungen bilden können.

Die durch Polymerisation hergestellten Polymergele werden dabei vorzugsweise auf Bandtrocknern getrocknet. Dabei wird die Gelschicht infolge von Inhomogenitäten oft ungleichmäßig getrocknet, so dass entweder Teile des Polymergels übertrocknet werden müssen, worunter die Produktqualität leidet, oder Teile des Polymergels nur unvollständig getrocknet werden. Unvollständig getrocknete Polymerpartikel führen zu Problemen in den weiteren Prozeßschritten und müssen abgetrennt werden.

Verfahren unvollständig getrocknete Polymerpartikel bei der Trocknung zu vermeiden sind beispielsweise aus JP-A-06-73518, JP-A-06-107800, EP-A-0 497 623, EP-A-0 508 810 und EP-A-0 574 248 bekannt.

JP-A-06-73518 beschreibt ein Verfahren zur Trocknung von Hydrogelen auf einem Bandtrockner, bei dem die Trocknerleistung kontinuierlich an die aktuelle Schichtdicke der Gelschicht angepasst wird, wodurch der Anteil unvollständig getrockneter Polymerpartikel vermindert wird.

JP-A-06-107800 lehrt, dass die Übertrocknung und das Abtrennen unvollständig getrockneter Polymerpartikel vermieden werden kann, wenn bereits vor der Trocknung übergroße Polymergelpartikel entfernt werden.

EP-A-0 497 623 offenbart ein Verfahren, bei dem das Polymergel vor der Trocknung extrudiert wird.

EP-A-0 508 810 und EP-A-0 574 248 lehren die Verwendung eines speziellen Knetreaktoren als Polymerisationsreaktor, wodurch größere Polymergelpartikel vor der Trocknung vermieden werden.

Aufgabe der vorliegenden Erfindung war die Bereitstellung eines verbesserten Verfahrens zur Herstellung wasserabsorbierender Polymerpartikel, insbesondere eine einfache und kostengünstige Abtrennung von übergroßen Polymergelpartikeln (Grobanteil) vor der Trocknung.

Gelöst wurde die Aufgabe durch ein Verfahren zur Herstellung wasserabsorbierender Polymerpartikel, umfassend Polymerisation einer Monomerlösung oder -suspension, enthaltend
a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,

Klassierung und Trocknung des erhaltenen feuchten Polymergels, sowie Zerkleinerung des bei der Klassierung des feuchten Polymergels abgetrennten Grobanteils, dadurch gekennzeichnet, dass die Klassierung des feuchten Polymergels mittels mehrerer kaskadenförmig angeordneter Stangendecks in einem Schwingrahmen durchgeführt wird.

Ein Stangendeck ist eine Anordnung nahezu paralleler Stangen. Die Stangen sind in Längsrichtung geneigt, so dass sich das Polymergel in Längsrichtung über das Stangeneck bewegen kann. Partikel, die kleiner sind als der Abstand der Stangen können durch das Stangendeck nach unten fallen und werden gesammelt.

Die Stangendecks sind weiterhin kaskadenförmig angeordnet, d.h., so dass sich das Polymergel zum Ende des vorderen Stangendecks bewegt, von dort auf den Anfang des nächsten Stangendecks fällt, usw.

Durch die Abtrennung und Zerkleinerung des Grobanteils vor der Trocknung werden unvollständig getrocknete Polymerpartikel verhindert und die Effizienz der Trocknung verbessert.

Die Stangendecks sind vorzugsweise von 5 bis 11 °, besonders bevorzugt von 6 bis 10°, ganz besonders bevorzugt von 7 bis 9°, in Produktstromrichtung gegenüber der Horizontalen geneigt.

Die Stangen sind typischerweise bezogen auf die Produktstromrichtung am Anfang eingespannt, so dass die einzelnen Stangen am Ende in vertikaler Richtung schwingen können.

Der Schwingrahmen schwingt in der Vertikalen mit einer Amplitude von vorzugsweise 2 bis 7,5mm, besonders bevorzugt 2,5 bis 5,5mm, ganz besonders bevorzugt 3 bis 4,5mm, wobei die Amplitude am Maschinengehäuse gemessen wird.

Die einzelnen Stangen schwingen in der Vertikalen mit einer Frequenz von vorzugsweise 5 bis 50Hz, besonders bevorzugt 10 bis 25Hz, ganz besonders bevorzugt 12 bis 20Hz.

Über die Neigung, die Amplitude und die Frequenz kann die Verweilzeit auf den Stangendecks eingestellt werden, wobei eine längere Verweilzeit die Trennschärfe erhöht.

Die Stangen haben einen Durchmesser von vorzugsweise 2 bis 14mm, besonders bevorzugt 4 bis 12mm, ganz besonders bevorzugt 6 bis 10mm. Mit abnehmendem Durchmesser nimmt die mechanische Stabilität der Stangen ab. Mit steigendem Durchmesser sinkt der freie Querschnitt der Stangendecks.

Die lichte Weite zwischen den Stangen beträgt im Ruhezustand vorzugsweise 5 bis 25mm, besonders bevorzugt 10 bis 20mm, ganz besonders bevorzugt 12 bis 18mm. Ein vertikales verschränken der Stangen kann die Trennwirkung erhöhen.

In einer bevorzugten Ausführungsform der vorliegenden Erfindung sind die Stangen mit einem wasserabweisenden Material beschichtet, d.h. die Oberfläche der Stangen weist einen Kontaktwinkel gegenüber Wasser von vorzugsweise mindestens 60°, besonders bevorzugt von mindestens 80°, ganz besonders bevorzugt von mindestens 100°, auf.

Der Kontaktwinkel ist ein Maß für das Benetzungsverhalten einer Flüssigkeit, hier Wasser, gegenüber einer Oberfläche und kann mit üblichen Methoden bestimmt werden, beispielsweise gemäß ASTM D 5725. Ein niedriger Kontaktwinkel bedeutet eine gute und ein hoher Kontaktwinkel eine schlechte Benetzung.

Geeignete Beschichtungen sind beispielsweise fluorhaltige Polymere, wie Perfluoralkoxyethylen, Polytetrafluorethylen, Ethylen-Chlortrifluorethylen-Copolymere, Ethylen-TetrafluorethylenCopolymere und fluoriertes Polyethylen, insbesondere Perfluormethylvinylether-Tetrafluorethylen-Copolymere.

Die wasserabweisende Beschichtung verhindert Anbackungen an den Stangen und verbessert die Trennschärfe.

Die Temperatur des feuchten Polymergels bei der Klassierung beträgt vorzugsweise von 50 bis 100°C, besonders bevorzugt von 60 bis 90°C, ganz besonders bevorzugt von 70 bis 80°C.

Vorteilhaft wird die Klassierung des feuchten Polymergels zur Umgebung thermisch isoliert. Dadurch kann ein Abkühlen des feuchten Polymergels während der Klassierung verhindert werden. Zusätzlich kann Wasserdampf eingeleitet werden.

Durch die obengenannten Bedingungen können die für die Klassierung des feuchten Polymergels optimalen Temperaturen eingestellt werden.

Der Schwingrahmen mit den Stangendecks wird typischerweise mittels eines Unwuchtmotors angetrieben. Die Stoßrichtung des Unwuchtmotors bezogen auf die Horizontale beträgt vorzugsweise von 30 bis 60°, besonders bevorzugt von 40 bis 50°, ganz besonders bevorzugt von 43 bis 47°.

Der Durchsatz an feuchtem Polymergel bei der Klassierung beträgt vorzugsweise von 1.000 bis 4.500 kg/m²h, besonders bevorzugt von 2.000 bis 3.500 kg/m²h ganz besonders bevorzugt von 2.500 bis 3.000 kg/m²h. Die Trennschärfe durchläuft mit steigendem Durchsatz ein Optimum.

Im Folgenden wird die Herstellung der wasserabsorbierenden Polymerpartikel näher erläutert:

Die wasserabsorbierenden Polymerpartikel werden durch Polymerisation einer Monomerlösung oder-suspension hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Dioder Triacrylaten veresterten mehrfach ethoxylierten und/oder propoxylierten Glyzerine, wie sie beispielsweise in WO 2003/104301 A1 beschrieben sind. Besonders vorteilhaft sind Diund/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,2 bis 0,6 Gew.-%, jeweils bezogen auf Monomer a). Mit steigendem Vernetzergehalt sinkt die Zentrifugenretentionskapazität (CRC) und die Absorption unter einem Druck von 21,0 g/cm² durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; Deutschland) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren sind beispielsweise Knetreaktoren oder Bandreaktoren. Im Knetreaktor wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Bandreaktor wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das Polymergel wird dann üblicherweise mit einem Bandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA empfohlenen Testmethode Nr. WSP 230.2-05 "Mass Loss Upon Heating" bestimmt wird. Bei einer zu hohen Restfeuchte weist das getrocknete Polymergel eine zu niedrige Glasübergangstemperatur T_{g} auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%.

Das getrocknete Polymergel wird hiernach gemahlen und klassiert, wobei zur Mahlung mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 µm, besonders bevorzugt von 250 bis 600 µm, ganz besonders von 300 bis 500 µm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von größer 150 µm beträgt vorzugsweise mindestens 90 Gew.-%, besonders bevorzugt mindestens 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Dies geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 µm, beträgt vorzugsweise mindestens 90 Gew.-%, besonders bevorzugt mindestens 95 Gew.-%, ganz besonders bevorzugt mindestens 98 Gew.-%.

Der Anteil an Partikeln mit einer Partikelgröße von höchstens 600 µm, beträgt vorzugsweise mindestens 90 Gew.-%, besonders bevorzugt mindestens 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Hydroxid, Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat, Citrat und Lactat, möglich. Es sind auch Salze mit unterschiedlichen Gegenionen möglich, beispielsweise basische Aluminiumsalze, wie Aluminiummo-noacetat oder Aluminiummonolaktat. Aluminiumsulfat, Aluminiummonoacetat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl während als auch nach der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

In einer bevorzugten Ausführungsform der vorliegenden Erfindung werden die wasserabsorbierenden Polymerpartikel nach der thermischen Trocknung gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; USA) und Nara Paddle Cooler (NARA Machinery Europe; Frechen; Deutschland). Überdies können auch Wirbelschichtkühler eingesetzt werden.

Im Kühler werden die wasserabsorbierenden Polymerpartikel auf 20 bis 150°C, vorzugsweise 30 bis 120°C, besonders bevorzugt 40 bis 100°C, ganz besonders bevorzugt 50 bis 80°C, abgekühlt.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert. Vorteilhaft wird die Nachbefeuchtung im Kühler nach der thermischen Trocknung durchgeführt.

Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Die gemäß dem erfindungsgemäßen Verfahren hergestellten wasserabsorbierenden Polymerpartikel weisen einen Feuchtegehalt von vorzugsweise 0 bis 15 Gew.-%, besonders bevorzugt 0,2 bis 10 Gew.-%, ganz besonders bevorzugt 0,5 bis 8 Gew.-%, auf, wobei der Feuchtegehalt gemäß der von der EDANA empfohlenen Testmethode Nr. WSP 230.2-05 "Mass Loss Upon Heating" bestimmt wird.

Die gemäß dem erfindungsgemäßen Verfahren hergestellten wasserabsorbierenden Polymerpartikel haben einen Anteil an Partikeln mit einer Partikelgröße von 300 bis 600 µm von vorzugsweise mindestens 30 Gew.-%, besonders bevorzugt mindesten 50 Gew.-%, ganz besonders bevorzugt mindestens 70 Gew.-%.

Die gemäß dem erfindungsgemäßen Verfahren hergestellten wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der wasserabsorbierenden Polymerpartikel beträgt üblicherweise weniger als 60 g/g. Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

Die gemäß dem erfindungsgemäßen Verfahren hergestellten wasserabsorbierenden Polymerpartikel weisen eine Absorption unter einem Druck von 49,2 g/cm² von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Absorption unter einem Druck von 49,2 g/cm² der wasserabsorbierenden Polymerpartikel beträgt üblicherweise weniger als 35 g/g. Die Absorption unter einem Druck von 49,2 g/cm² wird analog der von der EDANA empfohlenen Testmethode Nr. WSP 242.2-05 "Absorption Under Pressure, Gravimetric Determination" bestimmt, wobei statt eines Drucks von 21,0 g/cm² ein Druck von 49,2 g/cm² eingestellt wird.

Die EDANA-Testmethoden sind beispielsweise erhältlich bei der EDANA, Avenue Eugene Plasky 157, B-1030 Brüssel, Belgien.

### Beispiele

### Beispiel 1

Durch kontinuierliches Mischen von entionisiertem Wasser, 50gew.-%iger Natronlauge und Acrylsäure wurde eine Acrylsäure/Natriumacrylatlösung hergestellt, so dass der Neutralisationsgrad 69,0 mol-% entsprach. Der Feststoffgehalt der Monomerlösung betrug 43,5 Gew.-%. Als Vernetzer wurde 3-fach ethoxiliertes Glyzerintriacrylat (ca. 85gew.-%ig) verwendet. Die Einsatzmenge betrug 0,32 Gew.-%, bezogen auf unneutralisierte Acrylsäure.

Zur Initiierung der radikalischen Polymerisation wurden 0,01 Gew.-% Ascorbinsäure, 0,12 Gew.-% Natriumperoxodisulfat und 0,002 Gew.-% Wasserstoffperoxid, jeweils bezogen auf unneutralisierte Acrylsäure, eingesetzt.

Zwischen dem Zugabepunkt für Vernetzer und den Zugabestellen für die Wasserstoffperoxid/Natriumperoxodisulfat wurde die Monomerlösung mit Stickstoff inertisiert.

Die Reaktion wurde in einem Reaktor vom Typ List ORP 250 Contikneter, (LIST AG; Arisdorf; Schweiz) durchgeführt. Der Durchsatz der Monomerlösung betrug 800 kg/h. Der Austragswehr war maximal geöffnet (Wehrhöhe 78 mm).

Das erhaltene Polymergel wurde in einem Gelbunker aufgestaut und mittels einer Austragsschnecke aus dem Gelbunker zur Weiterverarbeitung entnommen.

Das feuchte Polymergel hatte eine Temperatur von ca. 75°C und wurde mittels eines Stangen-Sizers vom Typ SV 0536 (Mogensen GmbH & Co. KG; Wedel; Deutschland) klassiert. Der Siebdurchsatz betrug 1224 kg/h. Der Stangen-Sizer hatte drei kaskadenförmig angeordnete Stangendecks mit einer Länge von 500mm und einer Breite von 500mm. Die Stangendecks waren jeweils um 8° gegenüber der Horizontalen in Produktstromrichtung geneigt. Die Stangen hatten einen Durchmesser vom 8mm und waren mit MFA® (Copolymer aus Tetrafluorethylen und Perfluorvinylmethylether) beschichter Edelstahl (Werkstoff 1.4571). Die lichte Weite zwischen den Stangen im Ruhezustand betrug 15mm. Die Stangen waren bezogen auf die Produktstromrichtung am Anfang befestigt, so dass die einzelnen Stangen am Ende in vertikaler Richtung beweglich waren.

Die Stangendecks waren in einem Schwingrahmen befestigt und wurden mittels eines Unwuchtmotors angetrieben. Die Stoßrichtung des Unwuchtmotors bezogen auf die Horizontale betrug 45°. Der Stangen-Sizer schwang mit einer Amplitude von 3,7mm und einer Frequenz von 16Hz.

Das Gehäuse des Stangen-Sizers war thermisch isoliert. Zusätzlich wurden ca. 4,5 kg/h Wasserdampf mit einer Temperatur von ca. 120°C in den Stangen-Sizer dosiert.

Das mit dem Stangen-Sizer abgetrennte Polymergel (Grobanteil) wurde mittels eines Extruders vom Typ LSRE 75 R (Sela Maschinen GmbH; Harbke; Deutschland) zerkleinert. Der Extruder hatte 12 Löcher mit einem Durchmesser von 8mm.

Zur Überprüfung der Trennschärfe wurden 1,5 kg abgetrenntes Polymergel (Grobanteil) mit 200 g Sipernat D17 (Fällungskieselsäure) gemischt und einer Siebanalyse unterzogen. Der relative Anteil an Polymergel mit einer Partikelgröße von weniger als 15mm betrug 2,6 Gew.-%. Dieser Anteil gibt an wie viel Gutprodukt zusammen mit dem Grobanteil abgetrennt wird, bezogen auf die Gesamtmenge Gutprodukt im Zulauf. Idealerweise soll kein Gutprodukt zusammen mit dem Grobanteil abgetrennt werden. Die Trennschärfe der Klassierung ist daher umso besser, je kleiner dieser Anteil ist.

### Beispiel 2

Es wurde verfahren wie unter Beispiel 1. Der Austragswehr des Reaktors war maximal geschlossen (Wehrhöhe 170 mm). Der Siebdurchsatz betrug 1212 kg/h.

Der relative Anteil an Polymergel mit einer Partikelgröße von weniger als 15mm betrug 5,6 Gew.-%.

### Beispiel 3

Es wurde verfahren wie unter Beispiel 1. Es wurden Stangendecks mit unbeschichteten Stangen eingesetzt.

Nach wenigen Stunden war ca. 20% der Deckfläche mit Polymergel verklebt.

### Beispiel 4

Es wurde verfahren wie unter Beispiel 1. Der Stangen-Sizer schwang mit einer Amplitude von 5,2mm und einer Frequenz von 16Hz. Der Siebdurchsatz betrug 1260 kg/h.

Der relative Anteil an Polymergel mit einer Partikelgröße von weniger als 15mm betrug 6,9 Gew.-%.

### Beispiel 5

Es wurde verfahren wie unter Beispiel 1. Der Stangen-Sizer schwang mit einer Amplitude von 7,2mm und einer Frequenz von 16Hz. Der Siebdurchsatz betrug 880 kg/h.

Der relative Anteil an Polymergel mit einer Partikelgröße von weniger als 15mm betrug 36,4 Gew.-%.

### Beispiel 6

Es wurde verfahren wie unter Beispiel 5. Der Neutralisationsgrad der Acrylsäure/Natriumacrylatlösung wurde auf 75 mol-% und der Feststoffgehalt der Monomerlösung auf 40,7 Gew.-% eingestellt. Die Stangendecks des Stangen-Sizers waren jeweils um 9° gegenüber der Horizontalen in Produktstromrichtung geneigt. Der Siebdurchsatz betrug 912 kg/h.

Der relative Anteil an Polymergel mit einer Partikelgröße von weniger als 15mm betrug 11,2 Gew.-%.

### Beispiel 7

Es wurde verfahren wie unter Beispiel 6. Die Stangendecks des Stangen-Sizers waren jeweils um 11° gegenüber der Horizontalen in Produktstromrichtung geneigt. Der Siebdurchsatz betrug 424 kg/h.

Der relative Anteil an Polymergel mit einer Partikelgröße von weniger als 15mm betrug 35,4 Gew.-%.

### Beispiel 8

Es wurde verfahren wie unter Beispiel 1. Der Siebdurchsatz betrug 870 kg/h.

Der relative Anteil an Polymergel mit einer Partikelgröße von weniger als 15mm betrug 6,0 Gew.-%.

## Patentansprüche

1. Verfahren zur Herstellung wasserabsorbierender Polymerpartikel, umfassend Polymerisation einer Monomerlösung oder -suspension, enthaltend
a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,
Klassierung und Trocknung des erhaltenen feuchten Polymergels, sowie Zerkleinerung des bei der Klassierung des feuchten Polymergels abgetrennten Grobanteils, **dadurch gekennzeichnet, dass** die Klassierung des feuchten Polymergels mittels mehrerer kaskadenförmig angeordneter Stangendecks in einem Schwingrahmen durchgeführt wird.

2. Verfahren gemäß Anspruch 1, **dadurch gekennzeichnet, dass** die Stangendecks von 7 bis 9° in Produktstromrichtung gegenüber der Horizontalen geneigt sind.

3. Verfahren gemäß Anspruch 1 oder 2, **dadurch gekennzeichnet, dass** der Schwingrahmen in der Vertikalen mit einer Amplitude von 3 bis 4,5 mm schwingt.

4. Verfahren gemäß einem der Ansprüche 1 bis 3, **dadurch gekennzeichnet, dass** die Stangen in der Vertikalen mit einer Frequenz von 12 bis 20 Hz schwingen.

5. Verfahren gemäß einem der Ansprüche 1 bis 4, **dadurch gekennzeichnet, dass** die Stangen einen Durchmesser von 6 bis 10 mm aufweisen.

6. Verfahren gemäß einem der Ansprüche 1 bis 5, **dadurch gekennzeichnet, dass** die Stangen mit einem wasserabweisenden Material beschichtet sind.

7. Verfahren gemäß einem der Ansprüche 1 bis 6, **dadurch gekennzeichnet, dass** die lichte Weite zwischen den Stangen im Ruhezustand von 12 bis 18 mm beträgt.

8. Verfahren gemäß einem der Ansprüche 1 bis 7, **dadurch gekennzeichnet, dass** die Temperatur des feuchten Polymergels während der Klassierung von 70 bis 80°C beträgt.

9. Verfahren gemäß einem der Ansprüche 1 bis 8, **dadurch gekennzeichnet, dass** die Klassierung des feuchten Polymergels zur Umgebung thermisch isoliert ist.

10. Verfahren gemäß einem der Ansprüche 1 bis 9, **dadurch gekennzeichnet, dass** in die Klassierung des feuchten Polymergels zusätzlich Wasserdampf eingeleitet wird.

11. Verfahren gemäß einem der Ansprüche 1 bis 7, **dadurch gekennzeichnet, dass** der Schwingrahmen mittels eines Unwuchtmotors angetrieben wird.

12. Verfahren gemäß einem der Ansprüche 1 bis 8, **dadurch gekennzeichnet, dass** der Durchsatz an feuchtem Polymergel bei der Klassierung von 2.500 bis 3.000 kg/m²h beträgt.

13. Verfahren gemäß einem der Ansprüche 1 bis 12, **dadurch gekennzeichnet, dass** das Monomer a) zu mindestens 50 mol-% teilweise neutralisierte Acrylsäure ist.

14. Verfahren gemäß einem der Ansprüche 1 bis 13, **dadurch gekennzeichnet, dass** das Monomer a) zu 25 bis 85 mol-% neutralisiert ist.

15. Verfahren gemäß einem der Ansprüche 1 bis 14, **dadurch gekennzeichnet, dass** die wasserabsorbierenden Polymerpartikel eine Zentrifugenretentionskapazität von mindestens 15 g/g aufweisen.

## Claims

1. A process for producing water-absorbing polymer particles, comprising polymerization of a monomer solution or suspension comprising
a) at least one ethylenically unsaturated monomer which bears acid groups and may be at least partly neutralized,
b) at least one crosslinker,
c) at least one initiator,
d) optionally one or more ethylenically unsaturated monomers copolymerizable with the monomers mentioned under a) and
e) optionally one or more water-soluble polymers, classification and drying of the resulting moist polymer gel, and comminution of the coarse fraction removed in the classification of the moist polymer gel, wherein the classification of the moist polymer gel is performed by means of a plurality of bar decks arranged in a cascade in a vibrating frame.

2. The process according to claim 1, wherein the bar decks are inclined from 7 to 9° in product flow direction relative to the horizontal.

3. The process according to claim 1 or 2, wherein the vibrating frame vibrates in the vertical with an amplitude of 3 to 4.5 mm.

4. The process according to any of claims 1 to 3, wherein the bars vibrate in the vertical with a frequency of 12 to 20 Hz.

5. The process according to any of claims 1 to 4, wherein the bars have a diameter of 6 to 10 mm.

6. The process according to any of claims 1 to 5, wherein the bars have been coated with a water-repellent material.

7. The process according to any of claims 1 to 6, wherein the clearance between the bars at rest is from 12 to 18 mm.

8. The process according to any of claims 1 to 7, wherein the temperature of the moist polymer gel during the classification is from 70 to 80°C.

9. The process according to any of claims 1 to 8, wherein the classification of the moist polymer gel has been thermally insulated from the environment.

10. The process according to any of claims 1 to 9, wherein steam is additionally introduced into the classification of the moist polymer gel.

11. The process according to any of claims 1 to 7, wherein the vibrating frame is driven by means of an unbalance motor.

12. The process according to any of claims 1 to 8, wherein the throughput of moist polymer gel in the course of classification is from 2500 to 3000 kg/m²h.

13. The process according to any of claims 1 to 12, wherein monomer a) is acrylic acid partly neutralized to an extent of at least 50 mol%.

14. The process according to any of claims 1 to 13, wherein monomer a) has been neutralized to an extent of 25 to 85 mol%.

15. The process according to any of claims 1 to 14, wherein the water-absorbing polymer particles have a centrifuge retention capacity of at least 15 g/g.

## Revendications

1. Procédé pour la préparation de particules polymères absorbant l'eau, comprenant la polymérisation d'une solution ou d'une suspension de monomères, contenant
a) au moins un monomère éthyléniquement insaturé, portant des groupes acides, qui peut être au moins partiellement neutralisé,
b) au moins un réticulant,
c) au moins un initiateur,
d) éventuellement un ou plusieurs monomères éthyléniquement insaturés copolymérisables avec les monomères cités en a) et
e) éventuellement un ou plusieurs polymères solubles dans l'eau,
la classification et le séchage du gel polymère humide obtenu ainsi que le broyage de la fraction grossière séparée lors de la classification du gel polymère humide, **caractérisé en ce que** la classification du gel polymère humide est réalisée au moyen de plusieurs plans de barreaux disposés en forme de cascade dans un cadre oscillant.

2. Procédé selon la revendication 1, **caractérisé en ce que** les plans de barreaux sont inclinés de 7 à 9° par rapport à l'horizontale dans le sens d'écoulement du produit.

3. Procédé selon la revendication 1 ou 2, **caractérisé en ce que** le cadre oscillant oscille dans le plan vertical à une amplitude de 3 à 4,5 mm.

4. Procédé selon l'une quelconque des revendications 1 à 3, **caractérisé en ce que** les barreaux oscillent dans le plan vertical à une fréquence de 12 à 20 Hz.

5. Procédé selon l'une quelconque des revendications 1 à 4, **caractérisé en ce que** les barreaux présentent un diamètre de 6 à 10 mm.

6. Procédé selon l'une quelconque des revendications 1 à 5, **caractérisé en ce que** les barreaux sont revêtus d'un matériau repoussant l'eau.

7. Procédé selon l'une quelconque des revendications 1 à 6, **caractérisé en ce que** la lumière entre les barreaux à l'état au repos est de 12 à 18 mm.

8. Procédé selon l'une quelconque des revendications 1 à 7, **caractérisé en ce que** la température du gel polymère humide pendant la classification est de 70 à 80°C.

9. Procédé selon l'une quelconque des revendications 1 à 8, **caractérisé en ce que** la classification du gel polymère humide est isolée thermiquement par rapport à l'environnement.

10. Procédé selon l'une quelconque des revendications 1 à 9, **caractérisé en ce que** de la vapeur d'eau est en outre introduite dans la classification du gel polymère humide.

11. Procédé selon l'une quelconque des revendications 1 à 7, **caractérisé en ce que** le cadre oscillant est entraîné au moyen d'un moteur rotatif à balourd.

12. Procédé selon l'une quelconque des revendications 1 à 8, **caractérisé en ce que** le débit en gel polymère humide lors de la classification est de 2500 à 3000 kg/m²h.

13. Procédé selon l'une quelconque des revendications 1 à 12, **caractérisé en ce que** le monomère a) est, à raison d'au moins 50% en mole, de l'acide acrylique partiellement neutralisé.

14. Procédé selon l'une quelconque des revendications 1 à 13, **caractérisé en ce que** le monomère a) est neutralisé à raison de 25 à 85% en mole.

15. Procédé selon l'une quelconque des revendications 1 à 14, **caractérisé en ce que** les particules polymères absorbant l'eau présentent une capacité de rétention dans une centrifugeuse d'au moins 15 g/g.
